(19) Europäisches Patentamt
European Patent Office
Office européen des brevets

(11) Publication number: 0 370 960 A1

# (12) EUROPEAN PATENT APPLICATION

(21) Application number: 89830105.6

(22) Date of filing: 08.03.89

(51) Int. Cl.[5]: G01N 33/569, C07K 3/00, G01N 33/543, G01N 33/546

(30) Priority: 21.11.88 US 287133

(43) Date of publication of application: 30.05.90 Bulletin 90/22

(84) Designated Contracting States: AT BE CH DE ES FR GB GR IT LI LU NL SE

(71) Applicant: Pera, Ivo E.
312 McBrien Road Suite 5108
Chattanooga, TN 37411(US)

Applicant: Schur, Henry C.
145, Brownell Circle
Monterey, CA 93940(US)

(72) Inventor: Pera, Ivo E.
312 McBrien Road Suite 5108
Chattanooga, TN 37411(US)
Inventor: Schur, Henry C.
145, Brownell Circle
Monterey, CA 93940(US)

(74) Representative: Mannucci, Gianfranco, Dott.-Ing.
Ufficio Tecnico Ing. A. Mannucci Via della Scala 4
I-50123 Firenze(IT)

## (54) Neopterin as a marker in a diagnostic screening test kit to detect retrovirus diseases.

(57) This invention relates to diagnostic procedure for determining the presence or absence in the urine and/or plasma of NEOPTERIN which as a marker indicates an abnormal or pathological state or condition. More specifically, this invention is concerned with novel diagnostic reagents for immunological analysis of body substances.

EP 0 370 960 A1

Xerox Copy Centre

# NEOPTERIN AS A MARKER IN A DIAGNOSTIC SCREENING TEST KIT TO DETECT RETROVIRUS DISEASES

## BACKGROUND OF THE INVENTION

The clinical microbiology laboratory is responsible for the isolation and identification of microorganisms, i.e., bacteria, viruses, fungi, and protozoa, in specimens obtained from patients suspected of or diagnosed as suffering from infectious diseases.Ideally the microbiology laboratory should make a tentative identification of infecting organisms rapidly enough so that attending physicians can institute therapy or offer advice to eliminate potential spread of the disease. Unfortunately, until recently, isolation and identification of the causative agent, particularly when it is a virus, have been slow, and information obtained is retrospective, serving only to confirm the clinical diagnosis of the physicians. The process used for virus isolation is not only slow but expensive. Cultivation requires inoculation of virus-containing materials into living cell cultures, which require costly reagents and, most important, handling by highly trained and skilled personnel. For these reasons only about 4% of the approximately 12,000 clinical microbiology laboratories in the United States perform diagnostic virology tests.

Immunological techniques offer an alternative to cell culture in the diagnosis of viral disease, and recent developments in precision instrumentation and hybridoma technology are making possible reliable but costly test for the clinical laboratory.

This approach also allows the detection of viruses because they are present in-vivo in high concentrations. This is not true for most other viruses, and detection has been hampered by the lack of assays sensitive and specific enough to detect antigen in low concentrations, as immune complexes, and in the presence of interfering or nonspecific materials.

The importance of quick and inexpensive tests for the proper care of infected patients and for public health disease surveillance has stimulated the inventors to develop a rapid an inexpensive test kit for detecting retroviruses.

A number of pathological states or conditions in both human beings and animals, who can be, or often are diagnosed through the application of well-known immunological principles. It will be understood that the word "Animals" when employed herein includes "Human Beings" unless there is a specific statement to the contrary . Animals which are exposed to retroviruses increase urinary neopterin level. Neopterin is an intermediate in the biosynthesis of tetrahydroxy bioptyerin ($BH_4$) an essential cofactor for tyrosine and triptophan hydroxilation. $BH_4$ plays an important role in the biosynthesis of catecholamines and serotonin. Thus Neopterin has been shown to be a clinically useful biochemical marker for the detection of retrovirus diseases. Any protein which is not normally present in a given animal (in our case are hens) can, when introduced into such an animal under the proper conditions, engender the formulation of antibodies. All immunological testing procedures are based upon this so-called antigen-antibody reaction through the use of known diagnostic materials, in our case the Neopterin, to determine the presence or absence of the corresponding antibody or antigen in the test urine and or plasma.

When an antigen and its corresponding antibody come into contact under the proper conditions, they combine to form a complex which is less soluble than were either of the uncombined original components. This "insoluble" complex is discernible to the human eye in varying degrees. Certain antigens and antibodies will combine in a relatively short period of time to form large particles which are macroscopically visible. However, in many antigen-antibody systems the complex forms very slowly, and the particle size is so small that certain "carriers"must be employed to expedite the macroscopic visualization of the reaction. Among the carriers which have been employed are bentonite and latex particles. Such carriers are usually characterized as "indicators" for each diagnostic test.

Each of the several immunological diagnostic procedures which is employed in the art is characterized by a number of drawbacks or disadvantages, among which are lack of sensitivity, lack of specificity, lack of stability, lack of avidity (speed and quality of reaction), and improper character of the resultant test mixture to provide an easily ascertainable visual evaluation. In the case of certain antigen-antibody reactions, the principal drawback is the lack of sensitivity and specificity which often results in false positive reactions, thereby necessitating coincident controls to avoid any misinterpretation. Another principal drawback is the inordinate length of time required for the tests. This time factor has in the past precluded many physicians from utilizing the tests when elaborate laboratory facilities were not immediately available.

In the invention under subject the Diagnostic Test Kit for Neopterin is qualified as clinical tool in the evaluation of person at risk for HTLV and ARC.

If the suspected retrovirus disease is present, the resulting antigen-antibody reaction is generally manifested by precipitation or agglutination of the Neopterin complex.

## BRIEF DESCRIPTION OF THE INVENTION

It is an object of this invention to provide novel immunological diagnostic procedures and diagnostic reagents which are characterized by a high degree of sensitivity and specificity and which can provide accurate results in substantially faster time than previously was thought possible. Another object of this invention is to provide novel diagnostic procedures and reagents which can be routinely employed by a medical practitioner without the need of elaborate and costly laboratory facilities. Other objects and advantages, both general and specific, will appear as this specification proceeds.

The retrovirus screening test object of this invention, which, although not definitive to detect the type of retroviruse disease that affects the animal, is helpful in arriving at an immediate preliminary diagnosis.

The other test kits on the market are more difficult and time consuming and require special equipment or reagents, and demand a high degree of technical skill for the proper performance and interpretation. Through the use of the reagents of this invention, it is possible in minutes to determine whether a retrovirus condition is positive or negative, thereby facilitating proper corrective therapy.

The presence of the Neopterin in the urine is indicative that the animal is exposed to a Retrovirus disease.

A further object of the invention is to provide a specific immuno-diagnostic test using Neopterin as a biochemical marker for the clinical assessment of retroviruses, which will allow simple, sensitive, specific assays for detection of retroviruses within minutes. Furthermore, it is object of this invention to provide a person performing a diagnostic test with a simple container device containing antibodies against Neopterin, if Neopterin is present in the urine or serum, it combines with the antibodies causing a detectable change in the test reagent so that the test results can be visually observed. This will be a "positive" sign indicating that the patient is suffering from a retrovirus disease.

Still a further object of the invention is a method for the production of anti neopterin antibodies to be used in an immuno-diagnostic test which makes use of neopterin as a marker.

These and other objects will be clear to the skilled in the art after reading the following description.

In particular, the invention relates to a diagnostic procedure for the detection of retrovirus diseases, which is characterized in that a body fluid such as urine, serum, plasma or the like, containing biochemical neopterin is used in an antigen-antibody reaction, said biochemical neopterin acting as a marker for the detection of said retrovirus diseases.

In order to carry out the diagnostic procedure of the invention, the neopterin can be made to react with an anti neopterin antibody which is coupled onto and/or into a visualization enhancement compound or chemical. An anti neopterin antibody coupled compound can be used for detecting said neopterin.

The anti neopterin antibody used in the diagnostic procedure according to the invention may be used in an agglutination rest or a complemental fixation test as well.

As above mentioned, the present invention also refers to a method for the production of anti neopterin antibodies to be used in such a diagnostic procedure. According to the method of the invention, an immunogen carrying immunogenic determinants specific to elicit IgG anti neopterin antibodies is inoculated in fowls serving as hosts for active immunization thereof. The immunization of the fowls is carried out for a sufficient time in order to reach a desired concentration of antibody in the serum of the host. The anti neopterin antibodies are then collected from the egg yolk of the fowls. In practice, the immunization of the fowls can be carried out using a 6-(neopterin amino)-exanoic acid which is conjugated to bovin serum allumin (BSA) conjugate. The conjugate thus obtained is inoculated as an immunogen in the fowls for immunization thereof. The immunogen can be added to an adjuvant containing S.-typhimurium Re-mutant bacteria (STM) for enhancing the antibody response of the host to the immunogens. The method used for the separation of the anti neopterin antibodies from the yolk of the host's eggs are described in more detail in the following description and in the attached claims.

As above mentioned, the invention refers also to a screening test for a body fluid such as urine, serum or the like, wherein anti neopterin antibodies are used to detect the presence of clinically significant amounts of neopterin in said body fluid. In practice, the anti neopterin antibodies can be supported on a particulate support by adsorbtion technique, or by using a covalent coupling agent as well. In order to avoid self-agglutination of the antibodies, said antibodies may be disrupted in order to reduce their molecular weight and thus improve their resistance to self-agglutination.

In order to allow a better visualization of the test result, according to a further feature of the invention, the anti neopterin antibodies used in the screening test may be coated on the surface of a carrier. As a suitable carrier, latex particles may be advantageously used.

## GENERAL DESCRIPTION OF THE INVENTION

Retroviruses are unusual organisms that insinuate themselves into the life-styles of their hosts in revealing and often unprecedented ways: by converting their genes from RNA to a DNA form; by incorporating viral DNA stably into chromosomes of somatic or germ cells; by mutating, and even capturing, cellular genes; by rarely impairing, and often potentialing, the growth of their host cells; and by entrusting gene expression to host mechanisms under the direction of viral signals.
Retroviruses are popular in contemporary biology for many reasons, but in particular due to the recent discovery, that they are causative agents of the acquired immuno deficiency syndrome (AIDS), adult cell leukemia/lymphoma, herpes, mononucleosis and EBV syndrome.

Increased urinary Neopterin excretion has been observed in a variety of human diseases, including Acquired Immuno-Deficiency Syndrome (AIDS) and AIDS-Related Complex (ARC). The need for a simple, non-invasive screening test for infectious agents and AIDS especially prompted the inventors to develop a method and test kit for the diagnosis of retrovirus diseases, using Neopterin as biochemical marker.

Urinary Neopterin in the clinical assessment of AIDS, ARC and other retrovirus diseases has been used by the inventors for the development of the Virus Diagnostic Test Kit, object of this invention.
The measurement of urinary Neopterin and Biopterin is an important laboratory test for monitoring numerous pathological conditions. Abnormal levels of Biopterin and Neopterin may be present when there are alterations of pyrimidine, purine folic acid and other pteridine metabolic pathways.
Three important aromatic amino acid monooxygenases, phenylanine hydroxylase, tyrosine hydroxylase, and tryptophan hydroxylase are pterin dependent, and play an important role in the biosynthesis of biogenic amines, such as catecholamines and indolamines. Since these biogenic monoamines belong to the family of important intercellular messengers (i.e. neurotransmitters or hormones), changes in biopterin concentrations may have significant physiological and pathological effects. Urinary Neopterin have been shown to be significantly higher in ARC (AIDS related Complex) patients than normals, and significantly higher in AIDS patients than in normal patients. Urinary Neopterin assay will provide a quick, simple and safe aid for prognosis and for monitoring therapy.

Neopterin may be an in-vitro indicator of activation of the immune system. Its raised excretion has been related to the ac-tion of interferons on the T-lymphocyte/macrophage axis. It has also been suggested that an essential prerequisite for induction of Neopterin release in human mixed lymphocyte culture is T cell activation subsequent to the recognition of HLA-DR disparity. Increased urinary Neopterin levels have been observed in all patients with retroviral diseases and patients with AIDS.

High-performance liquid chromatography (HPLC) with fluorometry, bioassay by Crithida fasciculata, and radioimmunoas say have been used to assay Neopterin and Biopterin in human urine. HPLC is most frequently used. It is sensitive, and both Neopterin and Biopterin can be measured at the same time. However, it is time consuming for screening many samples.
Bioassay is sensitive and specific, but also time consuming. Radioimmunoassay is sensitive, specific and convenient for screening, but requires expensive reagents and involves the handling of radioactive materials. While costly laboratory test technique for measurement of Neopterin are known, no simple inexpensive diagnostic test kit available in the world market for use in doctor's office rather than in laboratory, before the invention under subject that can be used as a clinical tool in the evaluation of persons at risk for AIDS, ARC and other retroviruses.

During the year 1979/80 Radioimmuno assay for Biopterin in body fluids and tissues has been under investigation in Japan. Sugimoto Et Al., in Tokyo have published the following results:
"Specific antibodies against l-erythro-biopterin have been prepared in rabbits using the conjugates to bovine serum albumin. The antiserum against l-erythro-biopterin distinguished among l-erythro-tetrahydro- or 7.8-dihydro-biopterin, the other three stereoisomers of biopterin, D-erythro-neo-pterin, folic acid, and other synthetic pteridines. Using the specific antiserum against l-erythro-biopterin, a radio-immunoassay has been developed to measure the biopterin concentration in urine, serum, cerebrospinal fluid and tissue.
The conjugate of l-erythro-biopterin with tyramine, 4-hydroxy-2-[2-(4-hydroxyphenyl) ethylamino]-6-(1-crythro-1.2-dihydroxyptopyl)pteridine (BP-TYRA) was synthesized and labeled with $^{125}$I as the labeled ligand for the radioimmunoassay. BP-$^{125}$I-TYRA has similar binding affinity as the natural 1-erythro-biopterin and was thus permitted to establish a highly sensitive radioimmunoassay for biopterin. The limit of sensitivity of the radioimmunoassay with BP $^{125}$I-TYRA as labeled ligand was 0.5 pmol. The total concentration of biopterin i.e. biopterin, 7.8 dihydro, quinonoid dihydro and tetrahydrobiopterins in the biological samples was obtained by iodine oxidation under acidic conditions prior to the radioimmunoassay, whereas iodine oxidation under alkaline conditions gave the concentration only of the former two. Biopterin in urine could be measured directly using 1 ul of urine, but a pretreatment with a small Dowex 50-II column was required for serum, cerebrospinal fluid, and brain tissues.

The quantitative analysis of biopterin has been tried by using bioassay, radio-enzymatic assay based on

the cofactor activity for phenylanine hydroxylase, gas chromatography-mass fragmentography and high-performance liquid chromatography. Bioassay of biopterin for a growth factor of Crithidia fasciculata was the most frequently used, because of its high sensitivity. But it is time consuming and not exclusive of other pteridines possessing the growth factor activity such as 1-erythro-neopterin. The radioenzymatic assay is also fairly sensitive, but detects various tetrahydropterins which are active as a cofactor for phenylalanine hydroxylase, and the values obtained using the radioenzymatic method are known to be higher than those obtained by the bioassay. The other chemical methods are also time consuming. A gas chromatography mass fragmentography method requires the silylation of pterin and thus seems inapplicable for the assay of pterins in tissues. A recently reported procedure using high-performance liquid chromatography-fluorometry is sensitive and applicable for the analysis of pterins in urine blood, and tissue. But it is difficult to analyze many samples simultaneously by the chemical methods.

The radioimmunoassay of Sugimoto et al. has may advantages First, it is highly specific for the natural 1-erythro-biopterin, as compared with other methods such as bioassay, radioenzymatic assay or high-performance liquid chromatography. This radioimmuno assay can distinguigh even among the stereochemical isomers of biopterin, which cannot be distinguished by other methods. Although the result shows slight but significant cross-reactivity with some isomers, the potential for interference with the assay depends on the concentrations of these isomers in test fluids and tissues. Second, it is highly sensitive, and the total biopterin as well as reduced forms of biopterin in any body fluids and tissues can be assayed. Third, it is very simple and rapid. It was not necessary to preincubate the antiserum with 6,7-dimethylpterinylcaproyl-tyramide to remove antibodies against caproic acid moiety of the biopterin conjugate antigen to increase the sensitivity as in the previous method with BC-CAP-$^{125}$I-TYRA as the radiolabeled ligand. This can further simplify the radio immunoassay procedure. Samples as many as hundreds can be analyzed in a day, and this method is applicable to body fluids such as urine, serum, and cerebrospinal fluid and to any tissues.

It has been reported that the biopterin values obtained by this radioimmunoassay showed a fairly good agreement with the values obtained by bioassay. Biopterin concentrations in human urine and blood and in rat brain, obtained by this radioimmunoassay are also in good agreement with those by high-performance liquid chromotography. The concentrations of active biopterin cofactor, i.e., tetrahydrobiopterin plus quinonoid dihydrobiopterin can be measured as the difference between the values obtained by oxidation in acid give total biopterin (biopterin, 7,8-dihydrobiopterin), quinonoid dihydrobiopterin, and tetrahydrobiopterin) and those by oxidation in alkaline give only biopterin and 7,8-dihydrobiopterin, because tetrahydrobiopterin and quinonoia dihydrobiopterin convert to pterin but not to biopterin by alkaline oxidation.

This radioimmunoassay is very useful not only for biochemical, physiological and pharmacological studies on the pterin cofactor of aromatic amino acid hydroxylases, but also for the screening of a typical phenylketonuria due to biopterin deficiency and for the studies of pathogenesis of various diseases such as nervous and mental diseases where some abnormality in biopterin metabolism may occur. In fact, it has been found that in the striatum of parkisoniam human brain both biopterin concentrations and tyrosine hydroxylase activity were greatly reduced as compared with age-matched control human brains.

In accordance with prior art, it is known that birds, e.g. laying hens, transfer their immunity to the yolk of their eggs and thereby to their offspring. It is comparatively easy to raise and keep chickens under conditions where they will be exposed substantially only to the antigens against which immunity is desired. It is particularly advantageous that this immunity persists such long periods, such as the entire laying period without subsequent booster doses of antigen being necessary and the egg yolk antibodies are of high homogeneity, high purity, free from proleolytic enzimes, high stability, being substantially undamaged and free of dimers or higher aggregates.

The prior art related to immunological preparations, and techniques for putting this phenomenon to practical use, is described in several patents, particularly U.S. Patent #4.550.010 discloses certain preferred embodiment which can be combined with advantages for production, recovery and purification of antibodies from fowl eggs.

Immunological preparations are prepared by immunizing hens with an immugen having a molecular or particle weight of at least 30,000 to a stage of hyperimmunization at which there occurs a plateau-like levelling-off the antibody content of the serum. The immunogenicity of the immunogen can be enhanced by enlarging the immunogen particle mass. The eggs of the immunized hens are collected, the yolk is separated from the eggs, followed by separation of the lipid content of the yolk. The antibodies in the egg yolk are then rendered indispersable with the aid of a water-soluble linear filamentary non-charged polymer precipitant such as PEG and the indispersable antibodies are recovered. This precipitation of antibodies is advantageously preceded by a precipitation of caseinaceous proteins, lipid and yolk particles at lower polymer concentrations. Selected antibodies (IgY or IgG) can be concentrated by pH-controlled fractional

precipitation with PEG. The immunological preparations are useful for microassays and in appropriate cases also for the treatment or prophylaxis of pathological conditions.

Immunological preparations comprising antibodies can be put to a variety of uses, including the passive immunization of animals (including humans) and to an ever increasing extent, as immuno reagents for immunosorbtive processes and in particular for quantitative and qualitative analytical tests, in particular microassays for diagnostic, pathological, forensic and pharmacokinetic investigations.

Where the immunological preparations are used diagnostically, more particularly where an antigen used in the immunization of hens is associated with a pathological condition to be diagnosed, the antibodies separated in accordance with the invention, are applied to a pathological test sample and a precipitation reaction between the antibodies produced and the corresponding antigens in the test sample is observed in a manner which may be substantially as known per se. One technique is the well-known Ouchterlony technique, which requires no detailed description, since it is well-known in the literature and from standard textbooks. As a general rule it is preferred for the said technique to be carried out at a substantial sodium chloride concentration in the gel in which the test is carried out, which gel is preferably agarose gel. Suitable sodium chloride concentrations are generally between 0.1 and 0.2 M, usually 0. 15 M is preferred which is readily tolerated by the sensitive substances such as viruses. Higher concentrations are used only if no or no satisfactory precipitin reaction is observed in which case the concentration may be used to preferably not exceed 1.5 M.

This may yield better reaction with antigens of mol. weights less than 150,000.

A useful description of double diffusion techniques said in particular of the Ouchterlony method can be found in "Methods in Immunology and Immunochemistry" edited by C. H. Williams and Merrill W. Chase, Academic Press (1971), Vol. III pages 146-161.

Although the above reference recommends the use of either agar or agarose as gel medium for the test, agarose was found to be substantially more suitable in the context of the present invention because of white haloes formed around the yolk immunoglobulin (IgYO wells in agar (possibly due to a reaction product between agaro-pectin and residual traces of Lysozyme).

However, the Ouchterlony test, even when carried out at relatively high sodium chloride concentrations, is not preferred for use with IgY preparations according to the present invention when of particularly high uniformity as is readily attainable according to the invention as one of the advantages thereof. In that case the Ouchterlony technique may fail to produce a visible reaction. For that reason it is preferred to employ one of the numerous binding assays known in the art. The same applies in the case of determining relatively small haptens. Suitable techniques include radioimmunoassays and enzymes-linked immuno assays (ELISA). Also suitable are precipitation assays wherein the antibodies according to the present invention are used in combina tion with a further precipitant, e.g. ammonium sulphate or polyethyleneglycol or the like. The principles of those techniques are known to persons skilled in the art and require no further description.

The methods of enhancing in vivo antibody production to an antigen comprising cross-linking said antigen with an antiimmuno globulin to produce a conjugate thereof and then administering suitable amount of said-conjugate to a host are well known to people skilled in the art. A patent application has been filed in 1985 by the Dept. of Health , Inventors: J. Bersofsky and H. Kawamura, for a method of producing improved immune response to an antigen by targeting the antigen to the immune system. Features and many of the attendant advantages of the above invention will be better understood upon a reading of the following description. The advantages of the invention are achieved by proving a method of enhancing in vivo antibody production to an antigen comprising cross-linking said antigen with an antiimmunoglobulin to produce a conjugate thereof and then administering suitable amount of said conjugate to a host.

Immunogenicity of antigens in vivo or their potency in vitro is dependent on many factors including characteristics of the antigen itself and of the host. Relevant molecular properties of the antigen itself might include:

-a) size of the antigen, correlated with the number of antigenic determinants;

-b) affinity for functional structures of cells in the immune system, such as immunoglobulins (Ig) on B cells, cell surface membranes of antigen-presenting cells, Ia molecules; or

-c) susceptibility or resistance to lysosonal enzymes. In the whole animal distribution, catabolism and excretion of the injected antigen would influence the magnitude of immune reactions.

Several studies have shown that B cells can function as antigen-presenting cells.

Therefore, in an attempt to design an approach in which one can direct any antigen towards the immune system and see the effect on its immunogenicity, it was chosen to use Ig on B cells as the target of the antigen. Antigen bound to Ig on B cells should be presented to T cells efficiency. In this study, it is demonstated that ferritin, as antigen, coupled to antibodies to B cell surface Ig can be presented by whole

spleen cells to T-cells much more efficiently than free ferritin coupled to control Ig. In this case, cell separation experiments show that the enhanced presentation is primarily through B cells, not splenic accessory cells (SAC).

Furthermore, it was she that ferritin coupled to the correct Ig has greatly enhanced immunogenicity for antibody production in vivo. The simplest explanation for these effects is that the anti-IgM or anti-IgG (H + L) coupled to ferritin focuses the antigen with high affinity onto Ig receptors of B cells. The high affinity binding alone might account for the presentation at lower concentrations. These B cells then process and present ferritin to T cells. Also, the larger number of Ia-positive cells that can serve as presenting cells may decrease the threshold concentration for antigen to stimulate T cells.
Alternatively, the binding of anti-Ig to surface Ig on B cells may activate B cells to become more efficient nonspecific presenting cells similar to SAC.
Therefore targeting to B cells as antigen presenting cells, which because of their greater numbers and the increased affinity of binding via the anti-Ig antibody, increase the efficacy of antigen presentation to helper T cells. Then increased help results in increased antibody production

The eggs are collected after ten (10) days following the immunization injection for antibody extraction. Several methods for separating the Neopterin antibodies were compared, these involved the use of organic solvents, such as chloroform, ether, toluene and precipitation with ammonium sulphate. Best results were obtained by displacement with PEG.
Conventional method for the extraction of IgG with chloroform and ammonium sulfate is very simple. Start with: breaking egg into egg separator, rinse yolk with distilled $H_2O$, puncture yolk sac to drain yolk, discard sac, measure volume of yolk, add an equal volume of PBS, mix throughly, add twice the volume of chloroform, invert several times every 30 min. for 2 hours, let stand overnight (ON) at 4 C, centrifuge 2000 rpm 15 min., decant and save the upper saline layer, to this add an equal volume saturated ammonium sulfate, i.e. 50% $(NH_4)_2SO_4$, stir at 4 C for 30 min., let stand at 4 C for 2 hours, decant into centrifuge tubes, centrifuge N 5000xg/15 min./0 C, resuspend pellets in minimum volume, transfer to dialysis tubing, dialyse against saline for 18 h with 2 changes, transfer to 5 ml/serum vials, lyophilize until dry (i.e., freeze dry).

Polyethyleneglycol (PEG) Method: the method of separation of IgY is remarkable in its simplicity. All of the following is done at neutral pH (6.5-7.8) for which reason it is preferred to use a dilute phosphate buffer (0.1M) as the equeous medium throughout the process. The yolks collected from a number of eggs were throughly washed in a weak jet of distilled water to remove all the albumen. The yolks were punctured and dropped into a large glass funnel supported on a measuring cylinder. The fall into the funnel causes the yolk sack to break and release the yolk which collects in the cylinder and the sack discarded. The volume of yolk was measured and a volume of buffer equivalent to two volumes of yolk was added and throughly mixed.
PEG which had been finely pulverized in a Waring blender was added to a final concentration of 3.5% by weight of polymer to volume of diluted yolk. The mixture was stirred until all the polymer was dissolved. The mixture was centrifuged in a centrifuge at 10000 rpm (12,000xg) for ten min. This operation caused the separation of three phases in the centrifuge tubes. These were, a yellow fatty layer on the surface, a clear supernatant layer occupying the largest volume and a semi-solid pliable layer of the bulk of the yolk and caseinous protein "pellet" representing approximately 1/2 of the total volume of substance in the centrifuge tube. The supernatant fluid with the fatty layer was carefully decanted into a funnel containing an absorbent cotton plug in the neck of the funnel. This plug filtered off the lipid layer that was decanted with the supernatant fluid. The volume of the clear filtrate was measured and pulverised, PEG was added by gentle stirring to a final polymer concentration of 12 g PEG in 100 ml yolk extract. At this concentration the PEG caused complete displacement of the IgY. A certain percentage of associated proteins notably A and B livitin coprecipitated with the IgY. The precipitate was centrifuged off at 1000 rpm in a centrifuge. The pellets were redissolved to the original volume inphosphate buffer and the IgY once more precipitated with 12% pulverised PEG and centrifuged. The pellets obtained were complacted by subjection to a centrifugal force at 10000 rpm and the exuded solution of PEG originally entrapped in the pellets was removed by suction. The pellets were compacted once more and residual PEG solution discarded. In this manner the polymer which contaminated the IgY was reduced to a level at which it could not interfere with the antibody-antigen reaction. The final pellets were dissolved in a volume of phosphate buffer equivalent to half the volume of yolk from which it was derived. The protein concentration in the final product was of the order of 6 mg/ml. Sodium oxide (0.01%) was added as a preservative. Sodium oxide may also be incorporated earlier, namely in the buffer used for diluting the yolk (see above) in 0.01% concentration. By dissolving the pellets in a smaller volume, more concentrated solutions may be obtained if desired.
Optionally the IgY may be freed of traces of PEG by precipitation of the IgY with half saturated ammonium

sulphate followed by centrifugation. The PEG forms a liquid phase in the aqueous ammonium sulphate phase, while the IgY forms a third phase on the bottom of the centrifuge tube.

For many years nonionic polymers have been used as precipitating agents for macromolecules in various separation procedures, the physicochemical explanation of their precipitating ability is often given in terms of steric exclusion of the macromolecules from the domains of polymers.

Experiments performed as conventional precipitin reactions have been fundamental for the understanding of many of the effects of polymers on immunological systems. This led to the use of polymeric material to intensify the precipitation in different gel techniques, which is now a widely accepted method. Following the breakthrough of immunoprecipitin analysis (AIP) in 1970, polymer enhancement could also be applied to that technique, which resulted in simplifications and economical analyses.

The important characteristics of the polymer are thus those determing the net-forming properties, e.g. molecular weight, configuration (brancing, thickness of polymer bundles, etc.), and concentration, when a stearic exclusion mechanism is at work, one can thus expect to find an influence related to the tightness of the polymer network and also to the size of the antigen-antibody complexes.

Theoretically, the size of the antigenantibody complexes can be changed by using different ratios of antigen to antibody, and by using different sized reactants or reactants with different affinities.

In the last few years, polyethylene glycol (PEG) has been of great value in saturation analyses, the PEG precipitation method, originally considered useful mainly for analysis of low-molecular weight substances has been extended also to analyses of high molecular weight substances.

The great advantage of the PEG precipitation method is that the proteins are not denaturated by precipitation at room temperature, nor is the procedure particularly sensitive to changes in ionic strength of the medium in the physiological region, there is however, a relatively strong influence of the pH in the medium, the efficiency of the fractionation increases with the molecular weight of the polymer.

Several gel techniques are used to achieve enhanced immunological reaction with polymers, the most common are:

-infusion technique, in which the polymer solution is added to the antiserum through after the antiserum had diffused into the gel;

-immersion technique, in which the gel plates are immersed into a polymer solution after the first precipitin bands have become visible;

-admixture technique, in which antigen or antiserum is mixed with polymer before analysis. This technique is valid only for proteins having the same mobility as the enhancing polymer;

-crossed and rocket immunoelectrophoresis technique consists of a combination of electrophoretic separation of proteins in agarose gel followed by electrophoresis that is run perpendicular to the first separation into an antibody containing gel;

-single radial immunodiffusion (SRI) technique, in which the antigen is diffused into a gel containing the antiserum and the concentration of the antigen is determined by the size of the precipitin ring that is formed.

In principle, different effects can thus be obtained by means of different polymer concentrations in liquid media. At low concentrations there is an increase in sensitivity, whereas the length of the calibration curve is not markedly expanded; at high concentrations no increase in sensitivity is found in the region of antibody excess but the length of the calibration curve is expanded.

From the point of view of clinical chemistry, the work on AIP from 1969 to 1979 meant a new era in the field of immunological analyses. Whereas protein analyses had taken hours or days to perform, they now took 20 to 30 min. without loss of sensitivity. However, there were problems with blank-producing substances normally existing in sera that caused a loss of sensitivity. Another drawback with AIP was the need for monospecific antisera. Using earlier techniques such as the SRI technique or the rocket immunoelectrophoretic technique, monospecificity was not always necessary and dispecific antisera were even preferred in order to perform two immunochemical analyses in one step. However, good monospecific antisera are now available, so this is no longer a big problem.

The introduction of polymer enhancement through the Nephelometric technique brought with it several advantages to AIP. By performing the immunological reaction in a medium containing PEG, the sensitivity increased at the same time the reaction time could be decreased. Everyone who has worked with a continuous flow system knows that it is troublesome to have reaction times of 20 to 30 min. If something happens during the run, it is often not observed by the operator until 20 to 30 min. later. To clean the system and to start again means that an hour of the analyzing time has been lost. When run in a polymer medium, a reaction time of a few minutes is obtained. This also means that the analysis can be run with higher precision and less antiserum. The rate of formation of HIg-G-antibody complexes using a stopped flow nephelometer in which measurement can be performed as early as 3 min. after mixing of the reactants.

When PEG is included in the medium results on the markedly enhancing effect of the polymer are confirmed. Using PEG an obvious lag phase before the nephelometric response started this lag phase was not visible in the absence of polymers. After the lag phase, which lasted approximately 5 min. the reaction rate increased markedly and proceeded much more rapidly than in the absence of the polymer. An obvious increase in light scattering intensity is found when the molarity of sodium chloride in the buffer was decreased. This salt dependency is also observed in the presence of the polymer and influence not only the intensity rate but also the time of the lag phase.
As stated earlier, there are some conditions that should be taken into consideration before a low or high polymer concentration is to be used.

Low concentration systems.

Comparing results obtained with AIP and SRI can be noted that IgM was seriously underestimated with AIP. This discrepancy could be resolved by incorporating PEG in the buffer. In the absence of PEG, IgM in normal sera obviously reacted at a slower rate than in the calibration standards, although a reaction time of 18 min. was used. In the PEG medium, the reaction was essentially complete after a reaction time of 5 min.

The nephelometric response of the antiserum-polymer mixture per se recorded (the reagent blank or the antiserum baseline). For most polymers tested, the response was higher than that of the antiserum alone.

High concentration systems.

One of the advantages of using a high concentration of polymers in the medium is that the antigen excess peaks cannot be misinterpreted. It has been stated many times that the advantage of using a continuous flow system is the appearance of the typical split antigen excess peaks. Everyone who has worked with the system knows that this is not always so, especially not when peaks are obtained at very high antigen excess. The reason for the splitting of the peaks is that the samples are subdivided in the continuous flow air sectioned stream. When an antigen excess peaks enters the stream subsamples at the beginning and end react in proportion to the antigen concentration, whereas subsamples in the middle give a lower light scattering value because of the high antigen concentration. At very high antigen excess, however, these two side peaks will show up differently and the detection of antigen ex cessis then impossible. In using a high polymer concentration the risk of missing an antigen excess peak is avoided. This should thus be of great value for analysis of proteins in body fluids that can be expected to contain highly varying protein concentrations, e.g., on the other side of a biological membrane that will normally retain almost all of the proteins but which has been damaged by disease. Examples of such body fluids are urine, cerebrospinal fluid, and amniotic fluid. It is self-evident that the concentrations of polymer added to the antiserum solution must be chosen with respect to the solubility properties of the antigen.

The saturation analysis term was introduced as a general term instead of radioimmunoassay since often neither radioactive isotopes nor immonological reagents are used. A variety of separation techniques are being used in saturation analysis involving differential migration, adsorption, immunological precipitation or fractional precipitation of the free and/or bound fraction and solid phase methods.

Publications on the use of polymers in saturation analysis have been sparse except in the field of fractional precipitation where a great number of papers are being published on the use of PEG for precipitation of the antibody-bound antigen.

-a) solid-phase methods

-b) fractional precipitation methods

In all variety of immunological techniques using non-ionic polymers, it seems that steric exclusion dominate as probable explanation of the effect. Finally, some examples of nonionic polymer effects in other biological system should be mentioned. Polymers have been found to affect serological and agglutinating reactions which are immunological but which are not so easily discusses in steric exclusion effects since one of the "Reactants" has a very large size. A number of examples of the steric exclusion effect by polymers which do not involve im munological reactions are: -Serology, other agglutination reactions, complement activity, and enzyme activity.

DETAILED DESCRIPTION OF THE INVENTION

The procedure to make Neopterin-protein conjugate for the production of antibodies as per Sugimoto et Al. is as follows:

Various 2-amino-4-hydroxypteridines and their 5,6,7,8-tetrahydro derivatives having an alkyl or polyhydroxyalkyl subsequent at the 6-position possess distinctive biological activities. For example, biopterin (2-amino-4-hydroxy-6-(L-erythro-1, 2-dihydroxypropyl)-pteridine) is a growth factor for Crithidia fasciculate, and the 5,6,7,8-tetrahydro derivative is a potent coenzyme for phenylalanine, tyrosine and tryptophan hydroxylases The relation between the structure of 6-substituted 5,6,7,8-tetrahydro-2-amino-4-hydroxypteridines and the coenzyme activities for tyrosine hydroxylase has been studied extensively by Nagatsu and his coworkers. However, the pteridiens used in their study vary only in the substituents attached to the pyrazine ring of the compounds except a few cases. It is of interest to investigate how the biological activities of such pteridines will be changed by modification in the pyrimidine rather than the pyrazine moiety. This paper describes the synthesis of 6- or 6,7-disubstituted 2-amino-4-hydroxypterides possessing a carboxyalkyl group attached to the 2-amino group. The choice to introduce a carboxyalkyl group rather than a simple alkyl group stemmed from anticipation that such polar group cause a stronger interaction of the pteridine with the biological systems. In addition, it is expected that further modification of the compounds at the carboxyl group, for example the formation of an amino linkage to polyamines or proteins, would widen the utility of these compounds.

-Sinthesis of N-(4-Amino-6-hydroxy-5-nitvoso-2 pyrimidanyl) amino acids: A mixture of 4-amino-6-hydroxy-2-methythio-5-nitroso pyrimidine (1) (10g) and 6-amino hexanoic acid (20g) in water (400g) was heated under reflux for one hour. The resulting solution was adjasted to pH 2-3 with formic acid and chilled.

Filtration and washing with water gave an orange powder (85g) of 6-(4-amino-6-hydroxy-5-nitroso-2-pyrimidinyl) amino hexanic acid, which was pure enough for further reactions. This compound has the formula (2a) indicated hereinafter.

-Synthesis of 6-4-hydroxy-6-(L-erythro-1,2,3-trihydroxypropyl)-2-pteridinyl amino hexanic acid (5a): A solution of 2a (4.3g) in 2M sodium hydroxide (60ml) was hydrogenated over palladium on carbon (5%,2g) at room temperature and atmospheric pressure until the calculated amount of hydrogen was absorbed. After acidifying with concentrated hydrochloroc acid (20ml), the catalyst was removed by filtration. The filtrate was adjusted to pH 3-4, diluted with methanol (100ml) and heated with L-arabinose phenylhydrazone (3.8g) under nitrogen and reflux for 2 hours. After cooling, the solution was adjusted to pH 8-9 with ammonia and stirred under bubbling air at room temperature for 20 hours. The orange solution was evaporated to dryness in vacuo and the residue was extracted with 1M ammonia (200ml). The extract was adjusted to pH 2-3 with formic acid and chromatographed on a Florisil column (4,5x40cm) using water as the elution solvent. The eluate was evaporated to dryness and the residue was extracted with hot water (100ml). The extract, on concentration to about 50ml and chilling, gave pale yellow prism (2.0g) of 5a, mp 186-190 C, dec. (from water) (Found: C, 46.55; H,5.84; N, 17.95%, Calc. for $C_{15}H_{21}N_5O_6H_2O$: C, 46.75; H, 6.02; N, 18.17%).The compound has the formula (5a) indicated hereinafter.

-Conjugation of NEO-CAP to BSA: 65 ul of Ethylchloroformate was added to a solution containing NEO-CAP (74mg) and triethylamine (126ul) in 2 ml of N, N-dinethylformamide at -5C. After stirring for 15 minutes, the mixture was added to a solution of bovine serum albumin (114 mg) in 2.5 ml of 0.25M NaOH at 0 C The mixture was stirred at 0 C for 1 hours and then at 25 C for 30 minutes, during which periods the solution was maintained at pH 9-10 by addition of 1M NaOH. Finally the solution was made strongly alkaline by addition of 1M NaOH (2 ml) and kept at 25 C for 30 minutes. The alkaline solution was placed in a celophane tube and dialyzed against running water for 24 hours. The tube was immersed in aqueous 3% sodium acetate (300ml) whose pH was brought to 4.5 to cause precipitation inside the tube. The precipitate was collected by centrifugation and lyophilized to give the 6-(neopterin amino) hexanoic acid-bovine serum albumin conjugate (NEO-CAP-BSA) (95mg).

The synthesis summarized in the following scheme:

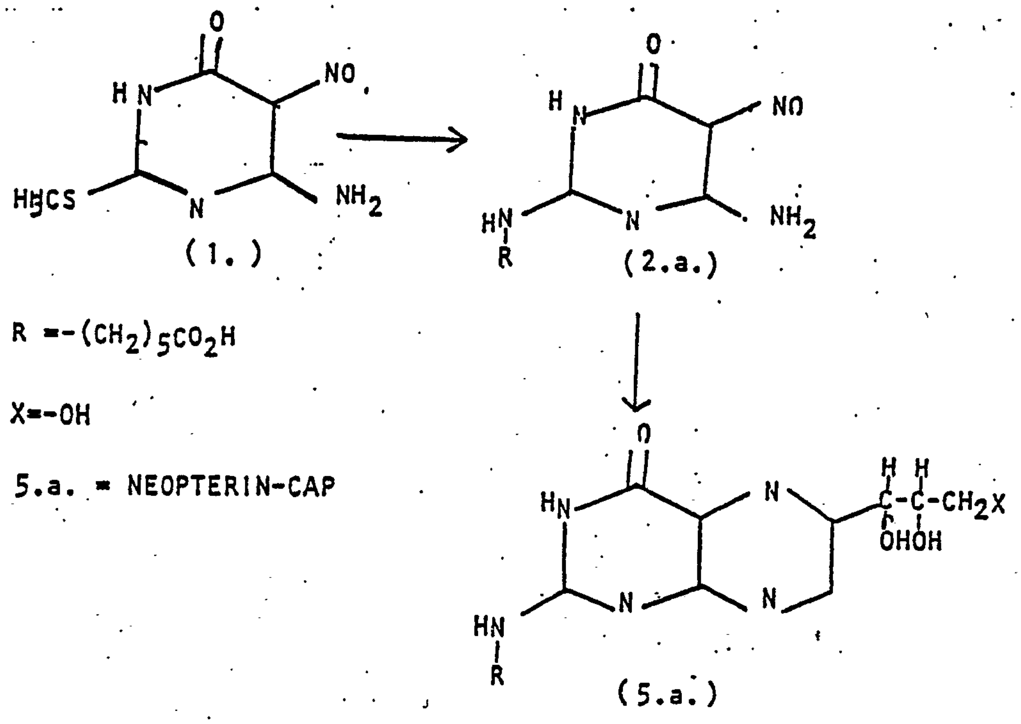

(1.)

(2.a.)

R = $-(CH_2)_5CO_2H$

X= -OH

5.a. = NEOPTERIN-CAP

(5.a.)

For the immunization procedure of laying hens using LES (Lipid emulsion system composed of lecitin, peanut oil and glycerin) and STM (S.typhimurium Re-mutant bacteria) as adjuvant

There are no fixed schedules for immunization of chickens with the antigens available, however, most studies done with the chickens have involved the use of either primary-response sera or antisera obtained after two injections of antigen. The procedures described here have been used by most investigators, and except for minor modifications, particularly in regard to production of antisera for study of the classes of immunoglobulins with antibody activity.

Most of the common breeds of chickens, and either males or females, may be used for antibody production. Although serological maturity is approached at about 9 weeks of age, older chickens are preferred for maximum production of antibodies. For most studies, antigen is administered either in the medial wing vein on the underside of a wing or in the jugular vein.

Prepare adjuvant STM in an aqueous, soluble, protein-atinous fraction isolated from S. typhimurium Re-mutant bacteria. STM is a mutogen for chicken lymphocytes and an adjuvant for enhancing the antibody response to antigens in fowl, LES is a completely metabolizable lipid emulsion system and is mixed with Dextran m/w 100.000.

Warm the vial containing LES-STM to 37C prior to use, to prepare an antigen add 3 volume of Neopterin-BSA or Neopterin-antichicken IgG antigen in saline, or PBS (3.75ml) to the LES + STM (1.25ml) vortes until a milky emulsion form. Each dose (0.5ml) will contain (1ng. Neopterin antigen will be used in the first immunization and 0.4ng of Neopterin antigen in subsequent booster injections) and 100ng of STM. Reconstituted vaccine can be stored at 0-4 C. for 2 weeks. Warm reconstituted vaccine to 37 C. and vortex prior to use.

Hens should be immunized every 4 days for the first week, then thereafter at an interval of 7 days, the dose will be administered by subcutaneous injections at several different sites on each hen (tail region and neck) as well as intramuscolary in the breast and tigh.

High concentration of antibodies are obtained within a week to ten days following the injection.

The test using standard Ouchterlony Method and other agglutination technique is an important factor in the characterization of the antibody is the titre. This can be expressed as the maximal amount of antigen precipitated by 1ml. of antibody. However, some confusion seems to exist, because it is not generally known that antibody titres, though expressed in apparently identical units, cannot be directly compared unless the titration methods are identical.

Using Ouchterlony Method: Pour a gel (3mm. deep layer of 1% agar in PBS) in plate. Punch holes in the agar using a rosett pattern as such as indicated with 1, 2, 3, 4, 5, 6 in the attached Fig. 1.

In the wells labelled 1-6, place 5-20 ul of a two (2) fold serial dilution of an antiserum of known

concentration or titer. Into the center well, place an equal aliquot of antigen. Cover the plate. Incubate 12-18 hr. a 37 C. A line of precipitation is indicative equal concentration of antigen antibody.
The following are other possible different titration methods where the antigen and antibody react in free solution.

Quantitative precipitin determination

The titration is performed by adding increasing amounts of antigen to a series of tubes containing a constant amount of antibody. The total volume in each tube is kept constant by the addition of saline.
After incubation at 37 C for 1 hour the tubes are refrigerated for at least 48 hours, the contents being mixed twice a day to ensure complete reaction between antigen and antibody.
The amount of protein precipitated is equivalent to the decrease of protein in the supernatant. The latter is estimated by reac tion with the Folin-phenol reagent. The antibody titre is calculated from the antibody and antigen amount in the tube with the largest amount of protein precipitated.

Sewell titration

The reaction between antigen and antibody takes place as described above. The supernatants are dispensed into wells punched in an agarose gel. Excess of antigen or antibody is directed by the ability of the supernatants to deflect a precipitation line formed between weels containing antigen and antibody, respectively. In the area of equivalent no deflection is seen.

Single radial immunodiffusion

Rocket immunoelectrophoresis. The single radial immunodiffusion and the rocket immunoelectrophoresis for the estimation of antibody titres are based on the fact that the area enclosed by a precipitation line is linearly related both to the amount of antigen and to the inverse of the antibody content in the gel. Let A1 $cm^2$ be the precipitation area produced by $x_1$ ug of antigen and A2 $cm^2$ the precipitation area produced by $x_2$ ug of antigen. If y is ml of antibody contained in 1 $cm^2$ of gel,then the titre T is defined by:

$$T = \frac{x_1 - x_2}{(A_1 - A_2)\, y} \quad ug/ml$$

Description of the preferred embodiments

The antibody may be supported on the support by a variety of techniques including adsorption; covalent coupling for example by activation of the support or by the use of a suitable coupling agent or by the use of the reactive groups on the support. Such procedures are generally known in the art, and no further details are deemed necessary for a complete understanding of the present test.
U.S. Patents N. 4,421,896 and N. 4,695,537 refer to techniques for supporting and antigen on a particulate support. These techniques may be also used for supporting an antibody according to the invention on a suitable particulate support. According to a preferred embodiment, the antibody is supported on the particulate support by an adsorption techinque, although other techniques may be used.
In accordance with preferred embodiment, the particulate support is either a polystyrene, aminated polystyrene, carboxylated polystyrene or a polyvinylchloride, although it is to be understood that the scope of the invention is not limited to such supports. As herein indicated, the antibody may be supported on the support by the use of an adsorption technique, or by covalent coupling agent. As representative examples of suitable coupling agents there may be mentioned: dialdehydes; for example glutaraldehyde, succinaldehyde, malonaldehyde, etc., unsaturated aldehyde, e.g., acrolein, methacrolein, crotonaldehyde, etc.;

carbodiimides, diisocyanates; dimethyladipimate; cyanur chloride, etc. The selection of a suitable coupling agent should be apparent to those skilled in the art from the teachings herein.

Similarly, the antibody may be supported by activation of a suitable support, for example cyanogen activated agarose.

The treatment of the purified virus with the surfactant is for a period of time sufficient di disrupt the virus and effect solubilization inerent, in general, such disruption and solubilization can accomplished in time periods in the order of from 5 to 120 minutes, however, in some cases longer or shorter times may be applicable.

The antibody which is treated and supported on a particulate support in accordance with the present test is preferably a viral antibody. The viral antibody may be recovered in accordance with a variety of procedures. The selection of an optimum treatment time is deemed to be within the scope of those skilled in the art from the teachings herein.

The surfactant or detergent which is used for treating the antibody may be one of a wide variety of surfactants or deter gents which disrupt the antibody, without destroying the antibody characteristics, including cationic, anionic and non-ionic surfactants. Such surfactants are well known in the art, and as representative examples, there may be mentioned alkali metal salts of sulfates, soaps, sulfates or sulfonated oils, various amines, quaternary salts, condensation products with ethylene oxide, etc. Preferred detergents for such use are alkali (lithium or sodium) dodecyl sulfate, sulfobetain, deoxylcholate and laurolylsarcosine (Sarcosyl).

As herein above indicated, it is believed that the antibody is disrupted so as to reduce its molecular weight and the detergent is preferably employed in an amount and for a time such as to effect such molecular weight reduction. In accordance with a preferred embodiment the molecular weight of the antibody does not exceed 200,000 daltons and most generally does not exceed 150,000 daltons, as determined by sucrose gradient centrifugation or exclusion chromatography.

In general the surfactant or detergent is employed in a weight ratio of surfactant to antibody of from about 0.05 to 1 to about 5 to 1. The selection of an optimum amount is deemed to be within the scope of those skilled in the art from the teachings herein.

After treating the antibody with the surfactant or detergent, the antibody may be supported on a solid particulate support. The sup-port may be any one of a wide variety of supports, and as repre-sentative examples of suitable supports there may be mentioned synthetic polymer supports, such as polystyrene, polypropylene, substituted polystyrene (e.g. aminated or carbohylated polystyrene), polyacrylamides, polyamide, polyvinylchloride, etc.; glass beads, agarose, cellulose; etc. The supports may be provided with reactive groups, e.g. carboxyl groups, amino groups, etc. to permit direct linking of the virus antibody to the support.

A wide variety of test kits are possible to take advantage of the advances in the diagnostic arts made possible by this inventions. Some will be described here, others can be devised by those skilled in the art.

The antibody reaction is the basis for all immunological test methods. Special proteins called antibodies are produced by an animal in response to the presence of an antigen, that is a foreign protein, in the body fluids of the animal. This normal body response to a foreign protein has led to the development of a number of techniques which are used to diagnose various human and animal diseases or disorders.

Immunological test methods may also be used to detect retroviruses. In vitro tests for the presence of a suspected antibody in a body fluid are carried out by adding the immunological counterpart to a vial of the body fluid. If the suspected protein is present the resulting antibody reaction is generally indicated by precipitation or agglutination of the antibody complex. As used herein the term body fluid refers to urine, serum, plasma or the like.

In some instances the antibody complex is slow to form and the particles that are formed are too small to be observed with certainty. In such cases, detectability of the antibody reaction can be improved by utilizing a carrier. When the antibody is coated on the surface of a carrier the reaction with the immunological counterpart produces a visible mass or agglutant. The proteinic antibody may be adsorbed onto the surface of carriers such as erythrocytes, bacteria cells, bentonite, polystyrene latex particles, anionic pherolic resins, or finely divided diazotized amino cellulose. It has been found however, that chemical binding of the antibody, molecule to the carrier is superior to physical adsorption.

Another test kit can use an agglutination immunoassay for antibody wherein antigen for sensitizing a solid particulate support is treated with detergent, in which the resultant antibody-sensitized support is resistant to self agglutination.

One aspect of the present test is that provides a solid support sensitized with an antibody preferably being derived from an intracellular parasite, in particular a virus.

More particularly, the antibody which is supported on the solid particle(s) (a particulate support) has been

treated with a detergent or surfactant so as to produce a particulate support sensitized with antibody which is more resistant to self-agglutination. Although applicant does not intend to be bound by any reasoning, it is believed that treating the antibody with the surfactant disrupts the antibody on the solid particles and such disrupted antibody on solid particles results in an increase in the resistance to self agglutination It is believed that the treatment disrupts the antibody and that such disruption may reduce the molecular weight which improves resistance to self-agglutination.

In accordance with a preferred embodiment, the antibody is treated with the detergent prior to being supported on the solid support; however, it is also possible to treat the antibody after being supported. Treatment on the support generally results in a loss in titer and, therefore is not preferred.
The antibody which are treated prior to being supported on the Particulate support are antibody which have a high molecular weight in excess of 500,000 daltons and in particular, antibodies from an intracellular parasite such as a retro-virus.
The treatment of the antibody with the detergent prior to being supported on the particulate support, may occur simultaneously with recovery of the antibody and prior to being supported on the solid particle(s)..
Thus, for example, a viral antibody may be recovered from intact virus by treating the virus with a detergent (surfactant) with disrupts the virus to provide the viral antibody and in such a case, the viral antibody may be supported on a solid particle to produce a sensitized particle which resists self-agglutination in that the viral antibody is treated with the detergent or surfactant prior to being supported on the solid support.

In the case where the antibody is recovered by a procedure which does not involve the use of a detergent or surfactant, then the recovered antigen is separately treated to disrupt the antibody prior to supporting the antibody on the solid particles so as to provide sensitized particles which resist self-agglutination.

The composition preparation and use of an antibody as chemical marker in a diagnostic test kit by the simple addition thereto of the urine or serum to be tested, is well known to those skilled in the art.
Therefore, the test kit devices of the invention under subject are not to be limited to placing the Neopterine marker in a test vessel and moistened with the liquid to be tested and then allowed to stand until agglutination or precipitin reaction does or does not occur, but may be extended to the preparation and use of a wide variety of immunological diagnostic test kits, which may employ different types of devices.

For the standardization of the test the basic step is to adjust the test reagent concentration such that more than 2 micrograms/deciliter of Neopterin will produce agglutination in the test system.
It is understood that the test kits described herein are for illustrative purpose only and that various modifications or changes in light thereof will be suggested to persons skilled in the art and are to be inclined within the spirit and purview of this application and the scope of the following claims.

## Claims

1. Immunological diagnostic procedure for the detection of retrovirus diseases, characterized in that a body fluid containing biochemical neopterin is used in an antigen-antibody reaction, said biochemical neopterin acting as a marker for the detection of said retrovirus diseases.

2. Immunological diagnostic procedure according to the preceding claim, characterized in that said neopterin is made to react with an anti neopterin antibody coupled onto and/or into a visualization enhancement compound or chemical.

3. Immunological diagnostic procedure according to claim 1, characterized in that an anti neopterin coupled compound is used for detecting said neopterin.

4. Immunological diagnostic procedure according to claims 1 to 3, characterized in that said anti neopterin antibody is used in an agglutination test to detect said neopterin.

5. Immunological diagnostic procedure according to the preceding claims, characterized in that said anti neopterin antibody is used in a complemental fixation test to detect said neopterin.

6. A method for the production of anti neopterin antibodies for the use in an immunological diagnostic procedure according to any of the claims 1 to 5, characterized in that an immunogen carrying immunogenic determinants specific to elicit IgG anti neopterin antibodies is inoculated in fowls serving as hosts for active immunization, and that after reaching a suitable antibody concentration in the serum of the host, the anti neopterin antibodies are collected from the egg yolk of the fowls.

7. Method for the production of anti neopterin antibodies according to claim 6, characterized in that a 6-(neopterin amino)-exanoic acid with the formula

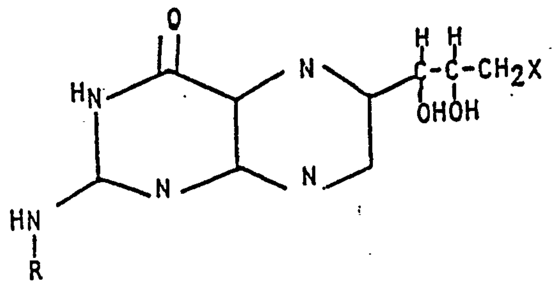

$R = -(CH_2)_5CO_2H$

is conjugated to a bovin serum albumin (BSA) conjugate to give a 6-(neopterin amino)-hexanoic acid-bovine serum albumin conjugate; and that said conjugate is inoculated as an immunogen in fowls for immunization thereof.

8. Method for the production of anti neopterin antibodies according to claim 6 or 7, characterized in that the immunogen is added to an adjuvant containing S.-typhimurium Re-mutant bacteria (STM) for enhancing the antibody response of the host to the immunogens.

9. Method for the production of anti neopterin antibodies according to claim 8, characterized in that the immunogen is added to an adjuvant containing STM and Lipid Emulsion System (LES) composed of lecitin, peanut oil and glycerin, the adjuvant and the immunogen being brought in an emulsion form before inoculation in the host.

10. Method for the production of anti neopterin antibodies according to claims 6 to 9, characterized in that for the separation of the anti neopterin antibodies from the yolk of the host's eggs a buffer such as a diluted phosphate buffer is added to the yolks, and to the mixture obtained polyethyleneglycole is added to cause precipitation of said anti neopterin antibodies; and that the polyethyleneglicol is finally removed from the final product.

11. Method for the production of anti neopterin antibodies according to claim 10, characterized in that said polyethyleneglycol is added to the yolks to a final concentration of 3,5% by weight of polymer to volume of diluted yolk.

12. Method for the production of anti neopterin antibodies according to claim 10 or 11, characterized in that the mixture of diluted yolk and polyethyleneglycole is stirred and centrifugated until separation of a supernatant fluid layer and a fatty layer, that said fatty layer and said supernatant layer are decanted until a clear filtrate is obtained; that said clear filtrate is pulverised and polyethyleneglycol added thereto until reaching of a concentration of 12g of polymer in 100 ml yolk extract in order to complete the displacement and the precipitation of the IgG containing the antibody.

13. Method for the production of anti neopterin antibodies according to claim 12, characterized in that the precipitated IgG containing the anti neopterin antibodies is redissolved in diluted phosphate buffer and once more precipitated with 12% polyethylenglicol and centrifugated for removing the polymer.

14. Method for the production of anti neopterin antibodies according to claim 13, characterized in that the IgG obtained in the second precipitation is dissolved in a phosphate buffer.

15. Method for the production of anti neopterin antibodies according to any of the claims 10 to 14, characterized in that sodium oxide is added to the solution containing the anti neopterin antibodies.

16. A screening test for a body fluid such as urine, serum and the like, characterized in that anti neopterin antibodies are used to detect the presence of clinically significant amounts of neopterin in said body fluid.

17. Screening test according to claim 16, characterized in that the anti neopterin antibodies are supported on a particulate support by adsorbtion technique.

18. Screening test according to claim 16, characterized in that the anti neopterin antibodies are supported on a particulate support by a covalent coupling agent.

19. Screening test according to claim 16, characterized in that the anti neopterin antibodies are disrupted antibodies having a reduced molecular weight and improved resistance to self-agglutination.

20. Screening test according to claim 19, characterized in that said anti neopterin antibodies are antibodies which are disrupted prior to be supported on the solid support.

21. Screening test according to claim 19 or 20, characterized in that the anti neopterin antibodies supported on the particulate support are antibodies treated with surfactant or detergent in a weight ratio of surfactant to antibody of from about 0.05: 1 to about 5.0:1.

22. Screening test according to any of the claims 16 to 20, characterized in that the anti neopterin antibodies are coated on the surface of a carrier.

23. Screening test according to claim 22, characterized in that the anti neopterin antibodies are

adsorbed onto the surface of latex particles.

Fig.1

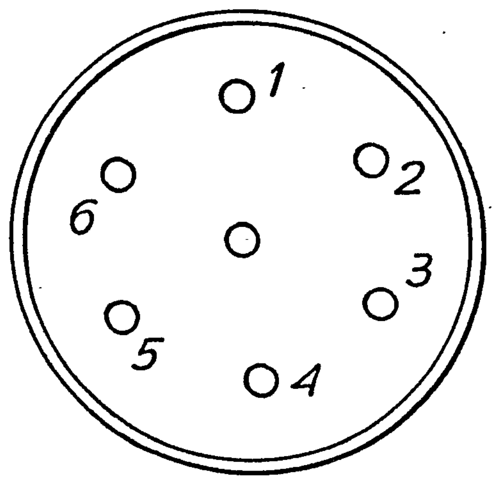

European Patent Office

# EUROPEAN SEARCH REPORT

Application Number

EP 89 83 0105

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 108, 25th April 1988, page 561, abstract no. 148434p, Columbus, Ohio, US; D. FUCHS et al.: "Neopterin as a marker in HIV infection", & CLIN. CHEM. (WINSTON-SALEM, N.C.) 1988, 34(2), 466-7 * Abstract * | 1,3 | G 01 N 33/569<br>C 07 K 3/00<br>G 01 N 33/543<br>G 01 N 33/546 |
| A | US-A-4 550 019 (A. POLSON) * Abstract; claims 1,6,28,52 * | 6,10,22 | |
| A | CHEMICAL ABSTRACTS, vol. 106, no. 15, 13th April 1987, page 457, abstract no. 117649s, Columbus, Ohio, US; H.D. VOLK et al.: "Neopterin determination and lymphocyte marker analysis with monoclonal antibodies: possibilities for determining immune-system activity for indicating diagnostic and therapeutic measures", & Z. EXP. CHIR., TRANSPLANT. KUENSTLICHE ORGANE 1986, 19(6), 351-4 * Abstract * | 1 | TECHNICAL FIELDS SEARCHED (Int. Cl.5)<br>G 01 N<br>C 07 N |
| A | CHEMICAL ABSTRACTS, vol. 105, no. 1, 7th June 1986, page 286, abstract no. 2920k, Columbus, Ohio, US; H. ROKOS et al.: "Determination of neopterin and reduced neopterins by radioimmunoassay", & BIOCHEM. CLIN. ASPECTS PTERIDINES 1985, 4(CANCER, IMMUNOL., METAB. DIS) 73-84 * Abstract * | 1 | |

The present search report has been drawn up for all claims

| Place of search | Date of completion of the search | Examiner |
|---|---|---|
| THE HAGUE | 12-12-1989 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document